# EUROPEAN PATENT SPECIFICATION

(11) **EP 2 155 068 B1**
(45) Date of publication and mention of the grant of the patent: **21.12.2016**
(21) Application number: 08755618.9
(22) Date of filing: 15.05.2008
(51) Int. Cl.: A61B 8/00, G01S 7/52, G01T 5/00, A61B 8/08, G06T 5/10, G06T 5/00, G01S 15/89

(54) **SYSTEM AND METHOD FOR ULTRASONIC HARMONIC IMAGING**
SYSTEM UND VERFAHREN ZUR HARMONISCHEN ULTRASCHALLDARSTELLUNG
SYSTÈME ET PROCÉDÉ POUR UNE IMAGERIE HARMONIQUE ULTRASONORE

(30) Priority: 16.05.2007 US 938371 P; 16.05.2007 US 938359 P; 16.05.2007 US 938446 P
(43) Date of publication of application: 24.02.2010
(73) Proprietor: Verathon, Inc., Bothell, WA 98011 (US)
(72) Inventor: WANG, Yanwei, Bothell, WA 98011 (US); MCMORROW, Gerald, Redmond, WA 98053 (US)
(74) Representative: Carter, Stephen John
(86) International application number: PCT/US2008/063803
(87) International publication number: WO 2008/144452

(56) References cited:
- WO-A2-2008/083386
- US-A- 5 473 555
- US-A- 5 526 816
- US-A- 5 820 559
- US-A- 5 873 829
- US-A- 6 106 465
- US-A- 6 146 330
- US-B1- 6 248 070

## Description

### FIELD OF THE INVENTION

Embodiments of the invention pertain to the image processing of targeted regions of interest scanned by ultrasound transceivers.

The invention is defined by the independent claims 1 and 5.

### BACKGROUND OF THE INVENTION

Ultrasound imaging depending on Fast Fourier Transforms (FFT) may lack the needed spectral information to generate diagnostically useful images under certain circumstances. The deficiency inherent in some FFT procedures can be overcome by using other approaches.

US patent application publication number US6106465A shows an ultrasonic method and system for boundary detection of an object of interest in an ultrasound image. An ultrasound imaging system that acquires an ultrasound image from a subject using harmonic information and automatically generates a boundary oriented with respect to an object of interest in the ultrasound image is disclosed. The ultrasound image used in this system can be generated with received ultrasonic energy at a fundamental frequency or at a harmonic of the fundamental frequency.

### SUMMARY OF THE PARTICULAR EMBODIMENTS

Systems and methods utilizing artificial intelligence via a harmonics analysis kernel (HAK) algorithm using returning first and second echo wavelength harmonics that arise from differential and non-linear wavelength distortion and attenuation experienced by transiting ultrasound energy returning from a targeted region-of-interest (ROI). The HAK algorithm is non-parametric and is substantially less susceptible to modeling errors. Using the harmonic ratios with a sub-aperture algorithm provides diagnostically useful images.

The sub-aperture algorithms are substantially fast enough to be implemented in real time within the time constraints enforced by ultrasound scanning protocols to acquire organ size information using scanning modalities besides the original ultrasound B-mode images. The harmonic information is collected using a long interrogating pulse with a single fundamental frequency. The received signal is collected, analyzed for its spectrum information about the first and second harmonics. The ratio of these two harmonics provides the quantitative information on how much harmonics have been generated and attenuated along its propagation. The sub-aperture algorithm may be executed in non-parametric mode to minimize data modeling errors.

### BRIEF DESCRIPTION OF THE DRAWINGS

The file of this patent contains at least one drawing executed in color. Copies of this patent with color drawing(s) will be provided by the Patent and Trademark Office upon request and payment of the necessary fee. Embodiments for the system and method to develop, present, and use clarity enhanced ultrasound images is described below.
FIGURES 1A-D depicts a partial schematic and a partial isometric view of a transceiver, a scan cone comprising a rotational array of scan planes, and a scan plane of the array of an ultrasound harmonic imaging system;
FIGURE 2A depicts a partial schematic and partial isometric and side view of a transceiver, and a scan cone array comprised of 3D-distributed scan lines in alternate embodiment of an ultrasound harmonic imaging system;
FIGURE 2B depicts a partial isometric view of an ultrasound harmonic bladder scanner system utilizing a transceiver probe and console combination; The object 77B is shown on display 16. A different set of arrows is shown on 77A but the format is a little different.
FIGURE 3 is a schematic illustration of a server-accessed local area network in communication with a plurality of ultrasound harmonic imaging systems;
FIGURE 4 is a schematic illustration of the Internet in communication with a plurality of ultrasound harmonic imaging systems;
FIGURE 5 schematically illustrates sub-algorithm processing algorithm to obtain harmonic profile smoothing;
FIGURE 6A is a raw data image of a bladder region of a patient;
FIGURE 6B is the ratio of the magnitude of the second harmonic to the magnitude of the first harmonic using an FFT of the raw data image of the bladder image of FIGURE 6A;
FIGURE 6C is the ratio of the magnitude of the second harmonic to the magnitude of the first harmonic frequency using the HAK of FIGURE 5 applied to the raw data image of the bladder image of FIGURE 6A;
FIGURE 7 is a panel of raw data images of a bladder region obtained from a patient;
FIGURE 8 are profiles of the ratio of the magnitude of the second harmonic to the first harmonic frequency based on the FFT of the respective raw data images of the patient's bladder regions of FIGURE 7;
FIGURE 9 are profiles of the ratio of the magnitude of the second harmonic to the first harmonic frequency based on the HAK of the raw data images presented in the B-mode scan of FIGURE 7;
FIGURE 10A depicts a panel of four second harmonic profiles of scan planes having theta angular values of 0, 15, 30, 45, 60, 75, 90, 105, 120, 135, 150, and 165 degrees;
FIGURE 10B depicts the twelve-second harmonic profiles of FIGURE 10A arranged or aligned in three-dimensional space;
FIGURE 10C depicts a simulated C-mode top view of the twelve-second harmonic parabolas projecting above the threshold plane;
FIGURES 11A-15A illustrates a series of 2D plot of the FFT processed second harmonics;
FIGURES 11B-15B illustrates the respective HAK companions of FIGURES 11A-15A in which the series of 2D plots are HAK processed second harmonics;
FIGURES 16A-B are regression analysis of harmonic ratio vs. bladder size plots of bladders not voided of urine;
FIGURES 17A-B are regression analysis of harmonic ratio vs. bladder size plots in of bladders after voiding of urine; and
FIGURES 18A-C are regression analysis of measured urine volume vs. HAK algorithm predicted volumes of a clinical group comprising 8 males and ten females.

### DETAILED DESCRIPTION OF THE PARTICULAR EMBODIMENTS

Particular embodiments described include a system and method to improve image clarity in ultrasound images that utilize an ultrasound transceiver receiving ultrasound energy returning from a targeted region of interest and producing a plurality of echoic signals. The region-of-interest may include an organ, an organ cavity, for example a bladder, or a portion of an organ or organ cavity. The echoic signals then receive signal processing via an executable algorithm configured to image the targeted region-of-interest from the echoic signals using at least one of a first harmonic, a second harmonic, and a fundamental frequency of the ultrasound energy. The algorithm generates a harmonic value that may then be plotted on a grid or render a map preventable on a computer display or other visual means. The executable algorithm includes a Harmonic Analysis Kernal (HAK). The HAK includes a window process, a Fast Fourier Transform process, an average process, a normalization of intensity process, a compensation-by-depth process, and a harmonic smoothing process to generate the harmonic values. A map of the harmonic values then may be coded, for example, by color-coding according to the magnitude of the harmonic value, to present an image of the region-of-interest.

Other systems, methods, and devices are configured for determining transducer functionality by using simulated body fluids, simulated body tissue, and combination simulated body fluids and body tissues for transducers having the characteristics of a 13 mm, 2.949 Mhz transducer in an ultrasound transceiver, for example the 9400 device developed by Verathon®, Inc. The transducer tests compare drive signal to produced signal in various environments (attenuating, non-attenuating, and a combination) and can also help us compare the produced signal to the received signal in the same various environments. These transducer tests help quantify the functionality of the 13mm transducers maximize the determination of transducer precision and accuracy.

The ultrasound transceivers or DCD devices developed by Verathon®, Inc. (formerly Diagnostic Ultrasound Inc.) are capable of collecting in vivo three-dimensional (3-D) cone-shaped ultrasound images of a patient. Based on these 3-D ultrasound images, various applications have been developed such as bladder volume and mass estimation. The clarity of images from the DCD ultrasound transceivers depends significantly upon the functionality, precision, and performance accuracy of the transducers used in the DCD ultrasound transceivers.

During the data collection process initiated by DCD, a pulsed ultrasound field is transmitted into the body, and the back-scattered "echoes" are detected as a one-dimensional (1-D) voltage trace, which is also referred to as a RF line. After envelope detection, a set of 1-D data samples is interpolated to form a two-dimensional (2-D) or 3-D ultrasound image.

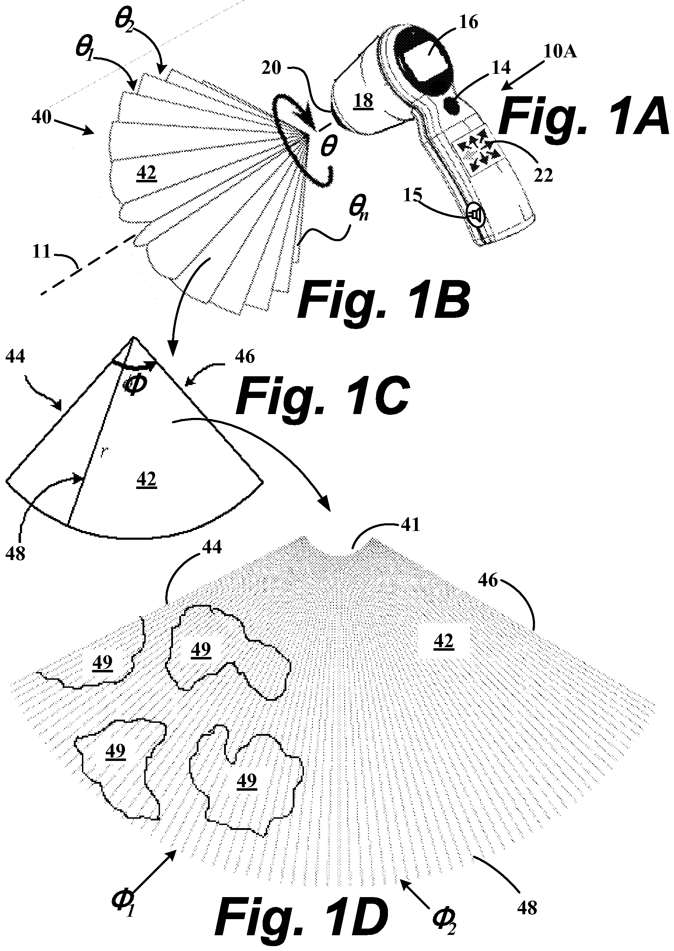

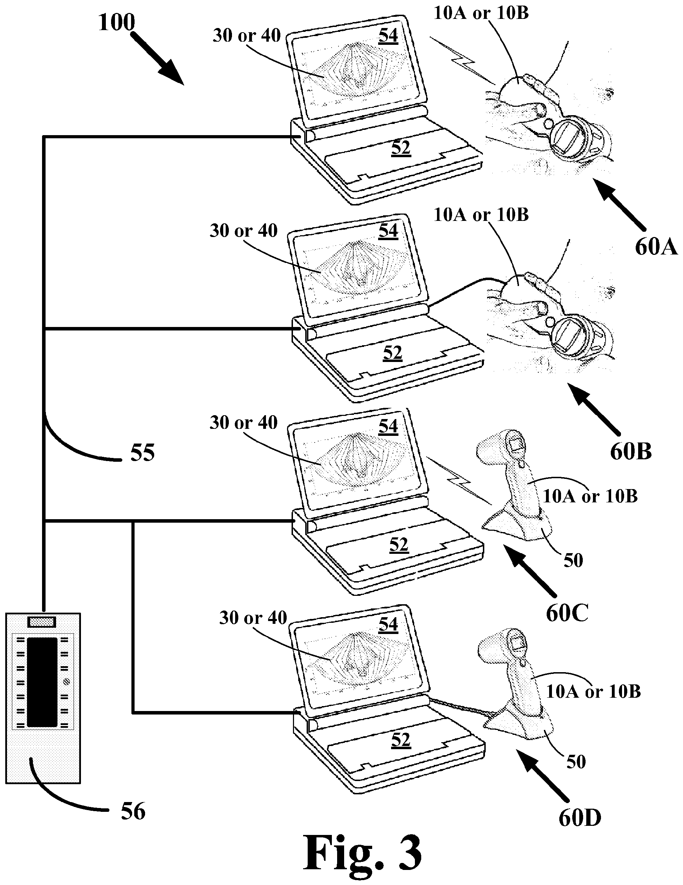

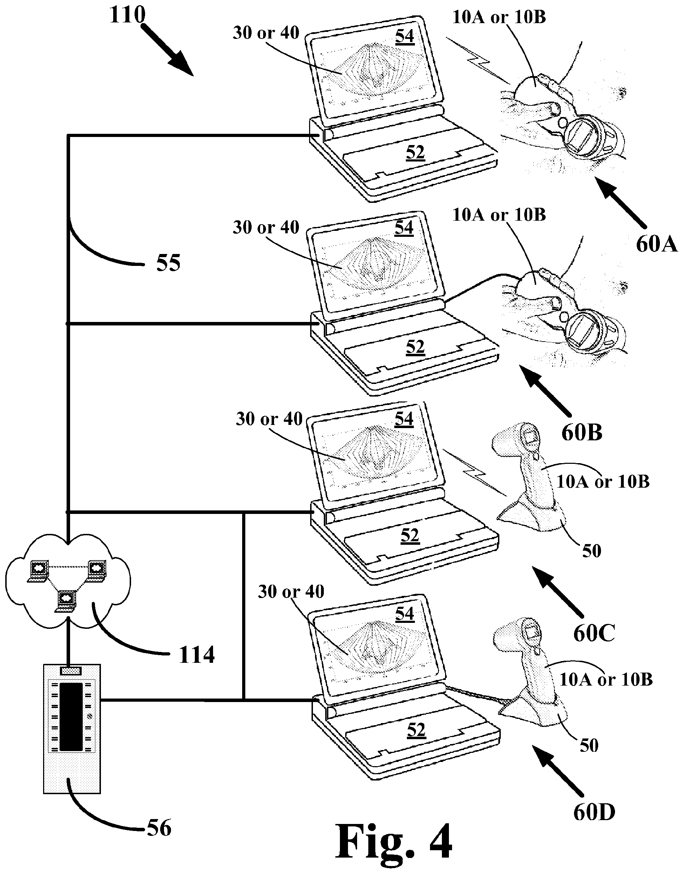

FIGURES 1A-D depicts a partial schematic and a partial isometric view of a transceiver, a scan cone comprising a rotational array of scan planes, and a scan plane of the array of various ultrasound harmonic imaging systems 60A-D capable of employing fundamental and/or harmonic ultrasound frequencies further illustrated in FIGURES 3 and 4 below.

FIGURE 1A is a side elevation view of an ultrasound transceiver 10A that includes an inertial reference unit, according to an embodiment of the invention. The transceiver 10A includes a transceiver housing 18 having an outwardly extending handle 12 suitably configured to allow a user to manipulate the transceiver 10A relative to a patient. Ultrasound transducers operating within the transceiver 10A may be equipped to collect and ready signals for ultrasound fundamental and/or harmonic frequency analysis.

The handle 12 includes a trigger 14 that allows the user to initiate an ultrasound scan of a selected anatomical portion, and a cavity selector (not shown). The transceiver 10A also includes a transceiver dome 20 that contacts a surface portion of the patient when the selected anatomical portion is scanned. The dome 20 generally provides an appropriate acoustical impedance match to the anatomical portion and/or permits ultrasound energy to be properly focused as it is projected into the anatomical portion. The transceiver 10A further includes one, or preferably an array of separately excitable ultrasound transducer elements (not shown in FIGURE 1A) positioned within or otherwise adjacent with the housing 18. The transducer elements may be suitably positioned within the housing 18 or otherwise to project ultrasound energy outwardly from the dome 20, and to permit reception of acoustic reflections generated by internal structures within the anatomical portion. The one or more array of ultrasound elements may include a one-dimensional, or a two-dimensional array of piezoelectric elements that may be moved within the housing 18 by a motor. Alternately, the array may be stationary with respect to the housing 18 so that the selected anatomical region may be scanned by selectively energizing the elements in the array.

A directional indicator panel 22 includes a plurality of arrows that may be illuminated for initial targeting and guiding a user to access the targeting of an organ or structure within an ROI. In particular embodiments if the organ or structure is centered from placement of the transceiver 10A acoustically placed against the dermal surface at a first location of the subject, the directional arrows may be not illuminated. If the organ is off-center, an arrow or set of arrows may be illuminated to direct the user to reposition the transceiver 10A at a second or subsequent dermal location of the subject. The acrostic coupling may be achieved by liquid sonic gel applied to the skin of the patient or by sonic gel pads to which the transceiver dome 20 is placed against. The directional indicator panel 22 may be presented on the display 54 of computer 52 in harmonic imaging subsystems described in FIGURES 3 and 4 below, or alternatively, presented on the transceiver display 16.

Transceiver 10A may include an inertial reference unit that includes an accelerometer 22 and/or gyroscope 23 positioned preferably within or adjacent to housing 18. The accelerometer 22 may be operable to sense an acceleration of the transceiver 10A, preferably relative to a coordinate system, while the gyroscope 23 may be operable to sense an angular velocity of the transceiver 10A relative to the same or another coordinate system. Accordingly, the gyroscope 23 may be of conventional configuration that employs dynamic elements, or it may be an optoelectronic device, such as the known optical ring gyroscope. In one embodiment, the accelerometer 22 and the gyroscope 23 may include a commonly packaged and/or solid-state device. One suitable commonly packaged device may be the MT6 miniature inertial measurement unit, available from Omni Instruments, Incorporated, although other suitable alternatives exist. In other embodiments, the accelerometer 22 and/or the gyroscope 23 may include commonly packaged micro-electromechanical system (MEMS) devices, which are commercially available from MEMSense, Incorporated. As described in greater detail below, the accelerometer 22 and the gyroscope 23 cooperatively permit the determination of positional and/or angular changes relative to a known position that is proximate to an anatomical region of interest in the patient. Other configurations related to the accelerometer 22 and gyroscope 23 concerning transceivers 10A,B equipped with inertial reference units and the operations thereto may be obtained from copending U.S. Patent Application Serial No. 11/222,360 filed September 8, 2005, herein incorporated by reference.

The transceiver 10A includes (or if capable at being in signal communication with) a display 16 operable to view processed results from an ultrasound scan, and/or to allow an operational interaction between the user and the transceiver 10A. For example, the display 24 may be configured to display alphanumeric data that indicates a proper and/or an optimal position of the transceiver 10A relative to the selected anatomical portion. Display 16 may be used to view two- or three-dimensional images of the selected anatomical region. Accordingly, the display 16 may be a liquid crystal display (LCD), a light emitting diode (LED) display, a cathode ray tube (CRT) display, or other suitable display devices operable to present alphanumeric data and/or graphical images to a user.

Still referring to FIGURE 1A, the cavity selector (not shown) includes a pressable button similar to the trigger 14 may be operable to adjustably adapt the transmission and reception of ultrasound signals to the anatomy of a selected patient. In particular, the cavity selector adapts the transceiver 10A to accommodate various anatomical details of male and female patients. For example, when the cavity selector is adjusted to accommodate a male patient, the transceiver 10A may be suitably configured to locate a single cavity, such as a urinary bladder in the male patient. In contrast, when the cavity selector is adjusted to accommodate a female patient, the transceiver 10A may be configured to image an anatomical portion having multiple cavities, such as a bodily region that includes a bladder and a uterus. Alternate embodiments of the transceiver 10A may include a cavity selector configured to select a single cavity scanning mode, or a multiple cavity-scanning mode that may be used with male and/or female patients. The cavity selector may thus permit a single cavity region to be imaged, or a multiple cavity region, such as a region that includes a lung and a heart to be imaged.

To scan a selected anatomical portion of a patient, the transceiver dome 20 of the transceiver 10A may be positioned against a surface portion of a patient that is proximate to the anatomical portion to be scanned. The user actuates the transceiver 10A by depressing the trigger 14. In response, the transceiver 10 transmits ultrasound signals into the body, and receives corresponding return echo signals that may be at least partially processed by the transceiver 10A to generate an ultrasound image of the selected anatomical portion. In a particular embodiment, the transceiver 10A transmits ultrasound signals in a range that extends from approximately about two megahertz (MHz) to approximately about ten MHz. Ultrasound energies beyond 10 MHz may be utilized.

In one embodiment, the transceiver 10A may be operably coupled to an ultrasound system that may be configured to generate ultrasound energy at a predetermined frequency and/or pulse repetition rate and to transfer the ultrasound energy to the transceiver 10A. The system also includes a processor that may be configured to process reflected ultrasound energy that is received by the transceiver 10A to produce an image of the scanned anatomical region. Accordingly, the system generally includes a viewing device, such as a cathode ray tube (CRT), a liquid crystal display (LCD), a plasma display device, or other similar display devices, that may be used to view the generated image. The system may also include one or more peripheral devices that cooperatively assist the processor to control the operation of the transceiver 10A, such a keyboard, a pointing device, or other similar devices. In still another particular embodiment, the transceiver 10A may be a self-contained device that includes a microprocessor positioned within the housing 18 and software associated with the microprocessor to operably control the transceiver 10A, and to process the reflected ultrasound energy to generate the ultrasound image. Accordingly, the display 16 may be used to display the generated image and/or to view other information associated with the operation of the transceiver 10A. For example, the information may include alphanumeric data that indicates a preferred position of the transceiver 10A prior to performing a series of scans. In yet another particular embodiment, the transceiver 10A may be operably coupled to a general-purpose computer, such as a laptop or a desktop computer that includes software that at least partially controls the operation of the transceiver 10A, and also includes software to process information transferred from the transceiver 10A, so that an image of the scanned anatomical region may be generated. The transceiver 10A may also be optionally equipped with electrical contacts to make communication with receiving cradles 50 as illustrated in FIGURES 3 and 4 below. Although transceiver 10A of FIGURE 1A may be used in any of the foregoing embodiments, other transceivers may also be used. For example, the transceiver may lack one or more features of the transceiver 10A. For example, a suitable transceiver need not be a manually portable device, and/or need not have a top-mounted display, and/or may selectively lack other features or exhibit further differences.

FIGURE 1B is a graphical representation of a plurality of scan planes that form a three-dimensional (3D) array having a substantially conical shape. An ultrasound scan cone 40 formed by a rotational array of two-dimensional scan planes 42 projects outwardly from the dome 20 of the transceivers 10A. Other transceiver embodiments of transceiver 10A may also be configured to develop a scan cone 40 formed by a rotational array of two-dimensional scan planes 42. The pluralities of scan planes 40 may be oriented about an axis 11 extending through the transceivers 10A-B. One or more, or preferably each of the scan planes 42 may be positioned about the axis 11, preferably, but not necessarily at a predetermined angular position *θ*. The scan planes 42 may be mutually spaced apart by angles *θ* 1 and *θ* 2. Correspondingly, the scan lines within each of the scan planes 42 may be spaced apart by angles *φ* 1 and *φ* 2. Although the angles *θ* 1 and *θ* 2 are depicted as approximately equal, it is understood that the angles *θ* 1 and *θ* 2 may have different values. Similarly, although the angles *φ* 1 and *φ* 2 are shown as approximately equal, the angles *φ* 1 and *φ* 2 may also have different angles. Other scan cone configurations are possible. For example, a wedge-shaped scan cone, or other similar shapes may be generated by the transceiver 10A.

FIGURE 1C is a graphical representation of a scan plane 42. The scan plane 42 includes the peripheral scan lines 44 and 46, and an internal scan line 48 having a length r that extends outwardly from the transceivers 10A. Thus, a selected point along the peripheral scan lines 44 and 46 and the internal scan line 48 may be defined with reference to the distance r and angular coordinate values *φ* and *θ*. The length r preferably extends to approximately 18 to 20 centimeters (cm), although any length is possible. Particular embodiments include approximately seventy-seven scan lines 48 that extend outwardly from the dome 20, although any number of scan lines is possible.

As described above, the angular movement of the transducer may be mechanically effected and/or it may be electronically or otherwise generated. In either case, the number of lines 48 and the length of the lines may vary, so that the tilt angle φ sweeps through angles approximately between -60° and +60° for a total arc of approximately 120°. In one particular embodiment, the transceiver 10 may be configured to generate approximately about seventy-seven scan lines between the first limiting scan line 44 and a second limiting scan line 46. In another particular embodiment, each of the scan lines has a length of approximately 18 to 20 centimeters (cm). The angular separation between adjacent scan lines 48 (FIGURE 1B) may be uniform or non-uniform. For example, and in another particular embodiment, the angular separation *φ* 1 and *φ*2 (as shown in FIGURE 1C) may be about 1.5°. Alternately, and in another particular embodiment, the angular separation *φ* 1 and *φ* 2 may be a sequence wherein adjacent angles may be ordered to include angles of 1.5°, 6.8°, 15.5°, 7.2°, and so on, where a 1.5° separation is between a first scan line and a second scan line, a 6.8° separation is between the second scan line and a third scan line, a 15.5° separation is between the third scan line and a fourth scan line, a 7.2° separation is between the fourth scan line and a fifth scan line, and so on. The angular separation between adjacent scan lines may also be a combination of uniform and non-uniform angular spacings, for example, a sequence of angles may be ordered to include 1.5°, 1.5°, 1.5°, 7.2°, 14.3°, 20.2°, 8.0°, 8.0°, 8.0°, 4.3°, 7.8°, and so on.

FIGURE 1D a graphical representation of a plurality of scan lines emanating from a hand-held ultrasound transceiver forming a single scan plane 42 extending through a cross-section of an internal bodily organ. The number and location of the internal scan lines emanating from the transceivers 10A within a given scan plane 42 may thus be distributed at different positional coordinates about the axis line 11 as may be required to sufficiently visualize structures or images within the scan plane 42. As shown, four portions of an off-centered region-of-interest (ROI) are exhibited as irregular regions 49. Three portions may be viewable within the scan plane 42 in totality, and one may be truncated by the peripheral scan line 44.

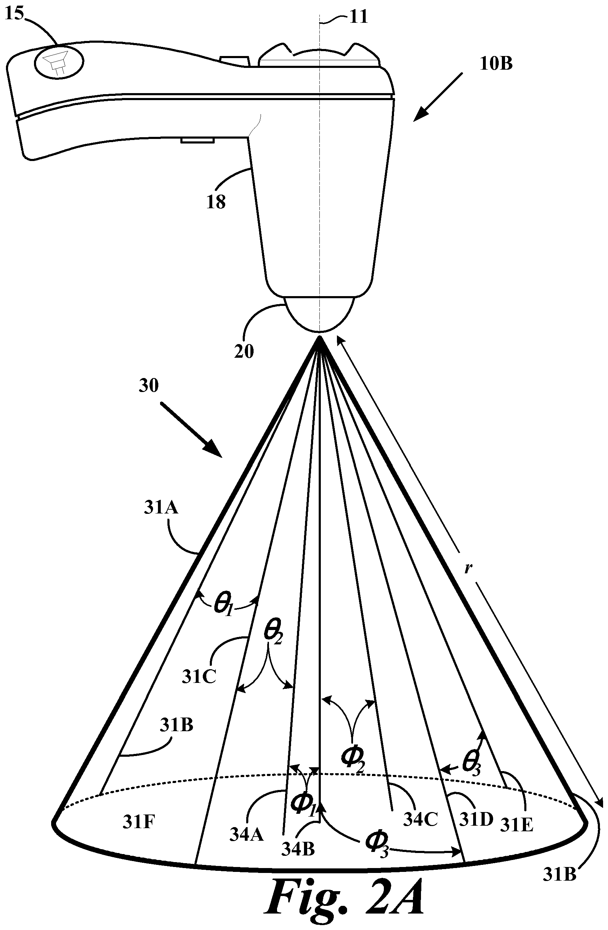

FIGURE 2A depicts a partial schematic and partial isometric and side view of a transceiver, and a scan cone array comprised of 3D-distributed scan lines in alternate embodiment of an ultrasound harmonic imaging system. A plurality of three-dimensional (3D) distributed scan lines emanating from a transceiver that cooperatively forms a scan cone 30. Each of the scan lines have a length r that projects outwardly from the transceivers 10A-10E of FIGURES 1A-1D. As illustrated the transceiver 10A emits 3D-distributed scan lines within the scan cone 30 that may be one-dimensional ultrasound A-lines. The other transceiver embodiments 10B-10E may also be configured to emit 3D-distributed scan lines. Taken as an aggregate, these 3D-distributed A-lines define the conical shape of the scan cone 30. The ultrasound scan cone 30 extends outwardly from the dome 20 of the transceiver 10A, 10B and 10C centered about an axis line 11. The 3D-distributed scan lines of the scan cone 30 include a plurality of internal and peripheral scan lines that may be distributed within a volume defined by a perimeter of the scan cone 30. Accordingly, the peripheral scan lines 31A-31F define an outer surface of the scan cone 30, while the internal scan lines 34A-34C may be distributed between the respective peripheral scan lines 31A-31F. Scan line 34B may be generally collinear with the axis 11, and the scan cone 30 may be generally and coaxially centered on the axis line 11.

The locations of the internal and peripheral scan lines may be further defined by an angular spacing from the center scan line 34B and between internal and peripheral scan lines. The angular spacing between scan line 34B and peripheral or internal scan lines may be designated by angle Φ and angular spacings between internal or peripheral scan lines may be designated by angle Ø. The angles Φ1, Φ2, and Φ3 respectively define the angular spacings from scan line 34B to scan lines 34A, 34C, and 31D. Similarly, angles Φ1, 02, and 03 respectively define the angular spacings between scan line 31 Band 31C, 31C and 34A, and 31D and 31E.

With continued reference to FIGURE 2A, the plurality of peripheral scan lines 31A-E and the plurality of internal scan lines 34A-D may be three dimensionally distributed A-lines (scan lines) that are not necessarily confined within a scan plane, but instead may sweep throughout the internal regions and along the periphery of the scan cone 30. Thus, a given point within the scan cone 30 may be identified by the coordinates *r*, Φ, and Ø whose values generally vary. The number and location of the internal scan lines emanating from the transceivers 10A-10E may thus be distributed within the scan cone 30 at different positional coordinates as may be required to sufficiently visualize structures or images within a region of interest (ROI) in a patient. The angular movement of the ultrasound transducer within the transceiver 10A-10E may be mechanically effected, and/or it may be electronically generated. In any case, the number of lines and the length of the lines may be uniform or otherwise vary, so that angle Φ sweeps through angles approximately between -60° between scan line 34B and 31A, and +60° between scan line 34B and 31B. Thus angle Φ in this example presents a total arc of approximately 120°. In one embodiment, the transceiver 10A, 10B and 10C may be configured to generate a plurality of 3D-distributed scan lines within the scan cone 30 having a length r of approximately 18 to 20 centimeters (cm).

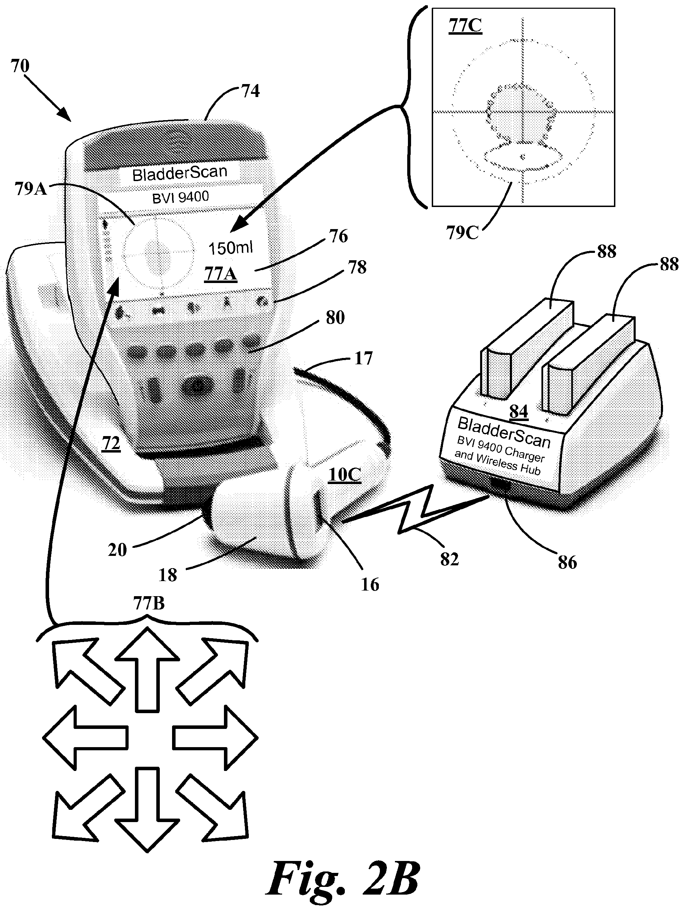

FIGURE 2B illustrates a partial isometric and schematic view of an ultrasound harmonic bladder scanner system 70 utilizing a transceiver probe 10C and console 74 combination 74. The harmonic bladder scanner system 70 is battery powered and portable and may also be referred to as the BVI9400 BladderScan system. Other embodiments may include line power. The ultrasound transceiver 10C is configured to detect and provide ultrasound harmonic echo signals to which the console 74 may image process using neural harmonic algorithms.

The transceiver 10C presents a similar transceiver display 16, housing 18 and dome 20 design as transceivers 10A and 10B, and is in signal communication to console 74 via signal cable 17. The console 74 may be pivoted from console base 72. The console 74 includes a display 76, detection and operation function panel 78, and select panel 80. The detection and operation function provide for targeting the bladder, allow user voice annotation recording, retrieval and playback of previously recorded voice annotation files, and current and previously stored 3D and 2D scans. In the display 76 is screenshot 76 having a targeting icon 79A with cross hairs centered in a cross sectional depiction of a bladder region. Other screen shots may appear in the display 76 depending on which function key is pressed in the function panel 78. A targeting icon screenshot 77B with a plurality of directional arrows may appear and flash to guide the user to move the transceiver 10C to center the bladder and can appear on either display 76 or display 16. The targeting icon screenshot 77B similar guides the user to place the transceiver 10C to center the bladder or other organ of interest as the directional indicator panel 22 depicted in FIGURE 1A above. An initial bladder view screenshot 77C may appear in which target icon 79A shows a central bladder region appearing within the cross hairs above the oval shaped pubic bone. In wireless communication via wireless signal 82, the output from the transceiver 10C may be outputted to a wireless hub 84 via wireless signal port 86. The wireless hub 84 also serves to charge batteries 88 for loading into the battery compartment (not shown) of console 74. All the calculations may be performed in the imaging console 74. The 9400 embodiment system 70 does not require a computer or network to complete the harmonic imaging processing. In other embodiments, the system 70 may utilize the wireless hub 84 as a gateway to transmit transceiver 10C acquired harmonic imaging information in local and Internet systems similar to those described in FIGURES 3 and 4 below.

FIGURE 3 is a schematic illustration of a server-accessed local area network in communication with a plurality of ultrasound harmonic imaging systems. An ultrasound harmonic imaging system 100 includes one or more personal computer devices 52 that may be coupled to a server 56 by a communications system 55. The devices 52 may be, in turn, coupled to one or more ultrasound transceivers 10A and/or 10B, for examples the ultrasound harmonic sub-systems 60A-60D. Ultrasound based images of organs or other regions of interest derived from either the signals of echoes from fundamental frequency ultrasound and/or harmonics thereof, may be shown within scan cone 30 or 40 presented on display 54. The server 56 may be operable to provide additional processing of ultrasound information, or it may be coupled to still other servers (not shown in FIGURE 3) and devices. Transceivers 10A or 10B may be in wireless communication with computer 52 in sub-system 60A, in wired signal communication in sub-system 10B, in wireless communication with computer 52 via receiving cradle 50 in sub-system 10C, or in wired communication with computer 52 via receiving cradle 50 in sub-system 10D.

FIGURE 4 is a schematic illustration of the Internet in communication with a plurality of ultrasound harmonic imaging systems. An Internet system 110 may be coupled or otherwise in communication with the ultrasound harmonic sub-systems 60A-60D.

The information obtainable from the scanning transceivers 10A or 10B used in sub-systems 60A-60D are derived from ultrasound echoes that are converted to signals received from structures in the body. These ultrasound echo signals carry not only the frequencies of the original transmit pulse, but also include multiples, or harmonics of these frequencies. These linear components are used in conventional, fundamental B-mode imaging.

In contrast, non-linear effects cause the harmonic echo frequencies during the propagation of ultrasound through various mediums. For example, THI (tissue harmonic imaging) is based on the phenomenon wherein ultrasound signals are distorted while propagating through tissue with varying acoustic properties. However, THI is merely an imaging method that does not solve the bladder detection problem.

Harmonic information is hidden in the frequency domain and it is an effective indicator for harmonic build-up on each scan line at different depth, based on which bladder lines and tissue lines can be separated. For example, inside a bladder region, there is not enough reflection, so the attenuations of the first and second harmonics are low. Deep behind the bladder wall, both the first and the second harmonics can be attenuated, while the second harmonic can be attenuated much faster than the first one. As a result, harmonic information can be higher for a scan line which passes through a bladder, compared to a scan line that penetrates tissue only.

One way to use the harmonic information is to use relative change of the harmonic information around the 2nd harmonic frequency compared with response at fundamental frequency. The ratio (Goldberg Number) of the peak value around the 2nd harmonic and the peak value around the fundamental frequency is a suitable indicator for such change.

From the clinical data collected from an ultrasound device, it can be observed that its spectrum is very noisy. This holds true even when there is little or no noise presented within the data. The convolution theory indicates that it is hard to use conventional FFT method to get good spectral estimation, not to mention that the stationary assumption does not hold for this data. A robust harmonic processing algorithm enables such a device to have good harmonic estimation results.

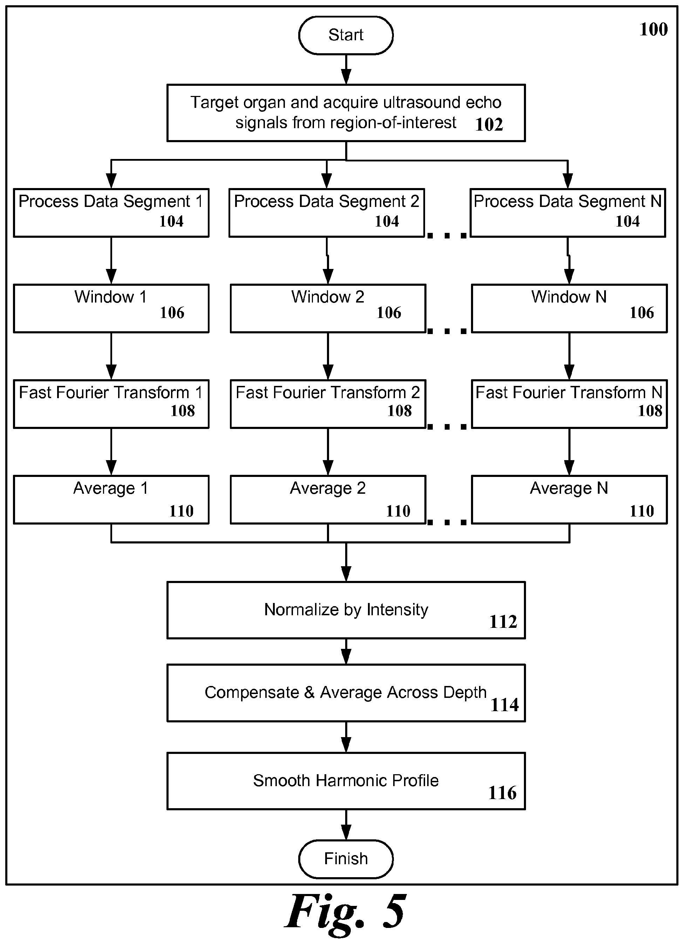

FIGURE 5 depicts a method flow chart of a Harmonic Analysis Kernel (HAK) 100 signal processing algorithm used to extract out information of a scanned region of interest of a subject. In general, such an approach may be based on sub-aperture processing technology, and it can be approximately regarded as a deconvolution process. The HAK 100 begins with process block 102 where a region-of-interest with the organ, or the whole organ, for example the bladder, is targeted with ultrasound transceivers 10A-B and ultrasound echoes are retrieved and converted to signals. The signals are subdivided along scan lines or a series of data segments from 1 to N. Thereafter, at block 104, the Data segments 1-N are processed. Each data segment 1 to N is independently processed by a Window block 106, a Fast Fourier Transform block (FFT) block 108, and an averaging processing block 110. After averaging, the pixel intensity is then normalized by intensity at process block 112, compensated and averaged across the depth of the scan line at process block 114, then outputted as a smoothed, harmonic profile at process block 116 to complete the HAK algorithm 100.

The window-processing block 106 utilizes a signal processing technique that applies a series of numerical weight to the echo signals resulting in a sub-signal set. The sub-signal set utilizes the Tailor window, a processing algorithm that allows computational adjustments between the mainlobe width and sidelobe levels common with signals exhibiting edge discontinuities. Thereafter, the FFT is applied at block 108, and the results thereof normalized with regard to the first harmonic spectrum intensity by taking the first harmonic spectrum intensity average and dividing it by the second harmonic spectrum intensity average. These calculations are then compensated with the expectation that a predicted attenuation of 2.5 dB/cm occurs to the imaging and/or echoic ultrasound energies.

The resulting data segments from the deconvolution process can be either overlapping or non-overlapping. For each data segment (on a single radio frequency (RF) pulse ultrasound data line), a Taylor window is applied to reduce its sidelobes from the Fast Fourier Transform FFT 108. After the FFT 108, an average of the spectrum around the first and the second harmonic frequencies is obtained at process block 110. Next, the normalization or compensation process is applied at block 112 to obtain an average the harmonic ratios based on the following sub-algorithm:

```
       Ratio_Sum = 0;
       Counter = 0;
       For each data segment(i)
       If (first harmonic>threshold)
       Ratio_SA(i) = 20*log10(first
       harmonic/second harmonic);
       Ratio_SA(i) = Ratio_SA(i) + i*Att_Comp;
       Ratio_Sum = Ratio_Sum + Ratio_SA(i);
       Counter = Counter +1;
       End if
       End for
       If (Counter >0)
       Ratio = Ratio_Sum/Counter;
       Else
       Ratio = Ratio_low
       End if
```

In the above sub-algorithm, 'Att_Comp' is an attenuation compensation parameter (a value of 2.5dB/cm can be used as and estimate from the clinical data). The 'threshold' is a parameter used to reject the data when they are too small. Ratio_low = -35dB. In summary, the 'normalization' step can remove the data segments that are too weak, the compensation step can compensate the harmonic ratio loss in tissue, and the averaging step can provide a more robust ratio estimator. The final step may be a spatial smoothing of the harmonic ratios across the scan lines within a scan plane.

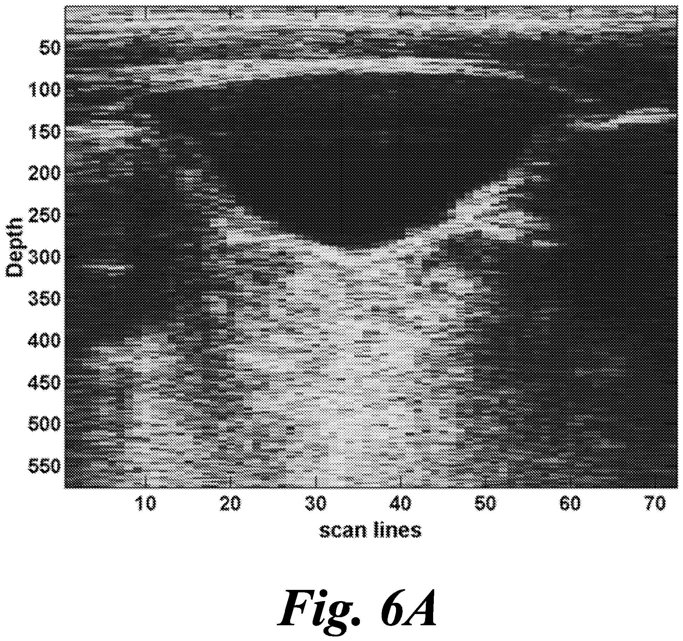

Clinical results obtained from a properly targeted scan in which the bladder is substantially centered are described in FIGURES 6A-C and FIGURES 7-9. A comparison is made of FFT and HAK processing of the scan results.

FIGURE 6A is a raw data image of a bladder region of a patient.

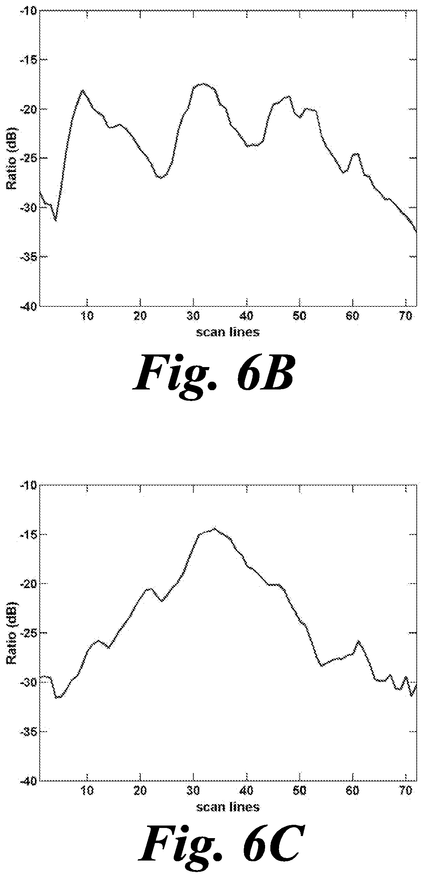

FIGURE 6B is the ratio of the magnitude of the second harmonic to the magnitude of the first harmonic using an FFT of the raw data image of the bladder image of FIGURE 6A

FIGURE 6C depicts a histogram of the ratio of the magnitude of the second harmonic to the magnitude of the first harmonic frequency using the HAK of FIGURE 5 applied to the raw data image of the bladder image of FIGURE 6A. Clearly the HAK's ratio is strongly correlated with the bladder, while the FFT has poor ratio estimates for the entire scan plane. According to this harmonic ratio model, the harmonic ratio estimated from each scan line can be related to the bladder size interrogated by this scan line.

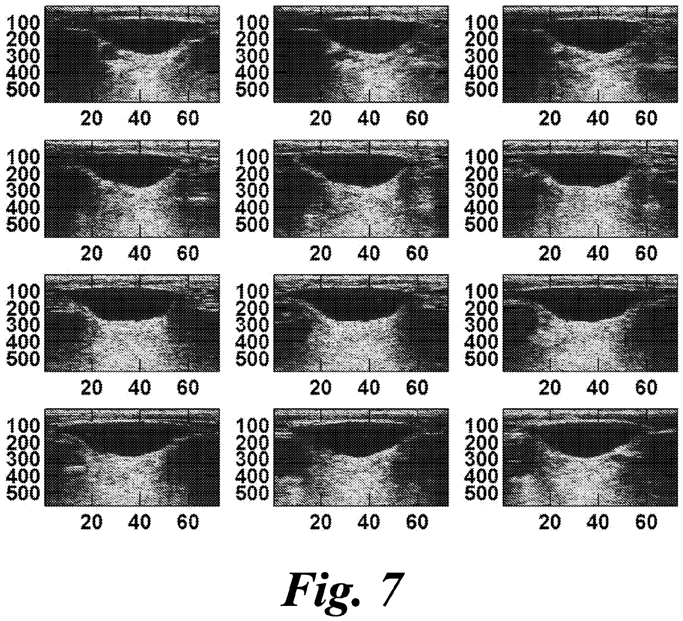

FIGURE 7 is a panel of raw data images of a bladder region obtained from a patient.

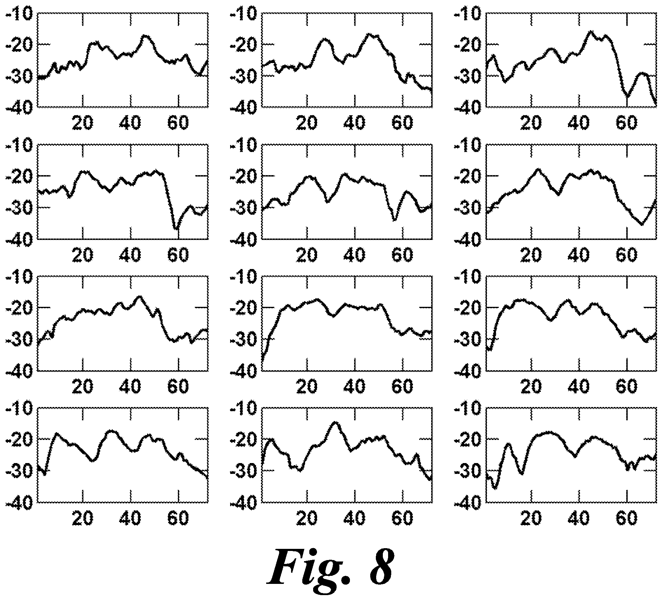

FIGURE 8 illustrate profiles of the ratio of the magnitude of the second harmonic to the first harmonic frequency based on the FFT of the respective raw data images of the patient's bladder regions of FIGURE 7.

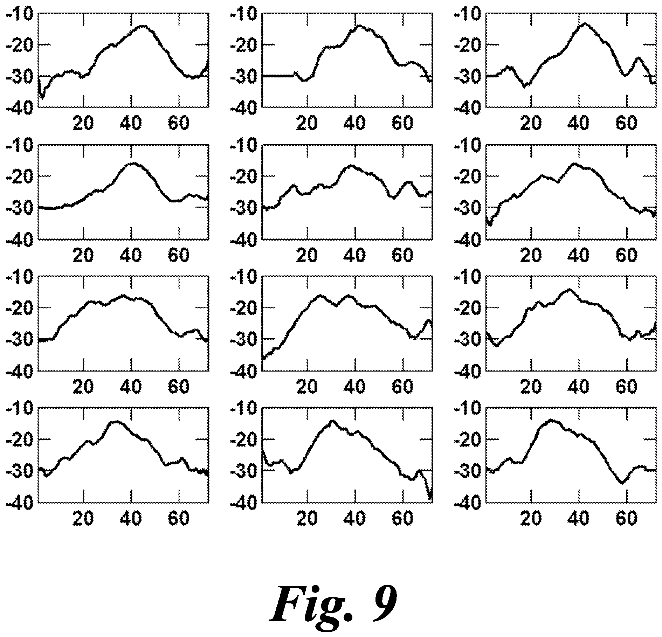

FIGURE 9 illustrates profiles of the ratio of the magnitude of the second harmonic to the first harmonic frequency based on the HAK of the raw data images presented in the B-mode scan of FIGURE 7. As with the HAK profiles of FIGURE 6C, these profiles obtained by the HAK algorithm of FIGURE 5 provide stronger correlations with the bladder depth, while the respective FFT of FIGURE 8 demonstrates poor ratio estimates for each scan plane.

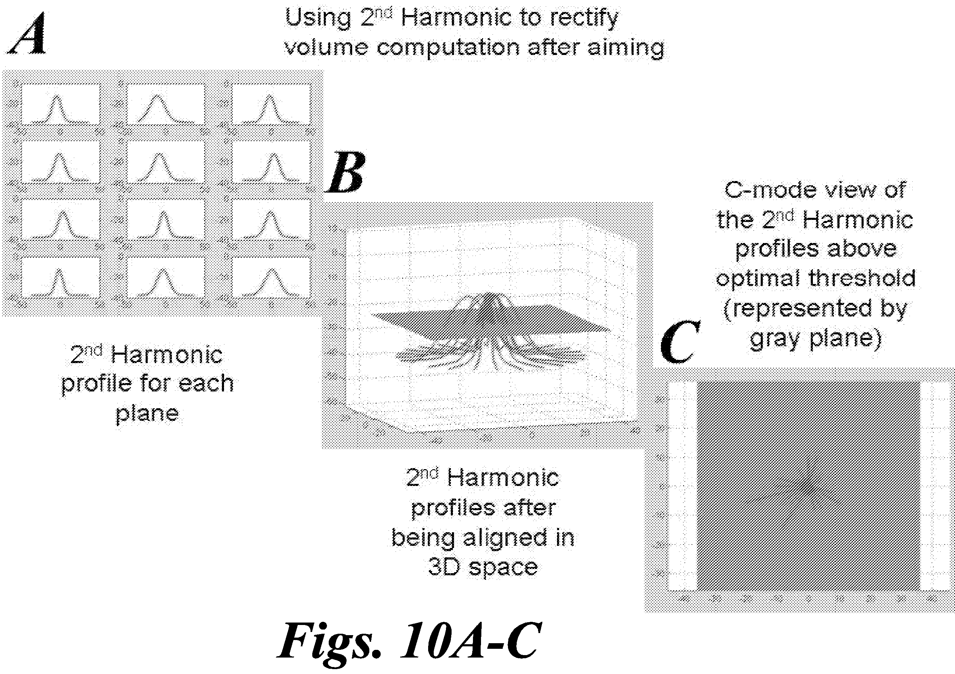

The second harmonics from the FFT and the HAK profiles may be plotted in 2-D presentations to enhance the visual delineation of a bladder region within a given scan plane via a color-coding process. The color-coding process is illustrated in FIGURES 10A-C.

FIGURE 10A depicts a panel of four second harmonic profiles of scan planes having theta angular values of 0, 15, 30, 45, 60, 75, 90, 105, 120, 135, 150, and 165 degrees. The second harmonic profiles present substantially parabolic and normally distributed patterns having varying distribution widths and peak maxima locations.

FIGURE 10B depicts the twelve-second harmonic profiles of FIGURE 10A arranged or aligned in three-dimensional space. The parabolic profiles of each scan plane may be arranged in a scan cone plane array similar to scan cone 40 of FIGURE 1B. The second harmonic parabolas rise above the threshold value represented as a gray plane.

FIGURE 10C depicts a simulated C-mode top view of the twelve-second harmonic parabolas projecting above the threshold plane. The threshold is represented in the gray plane.

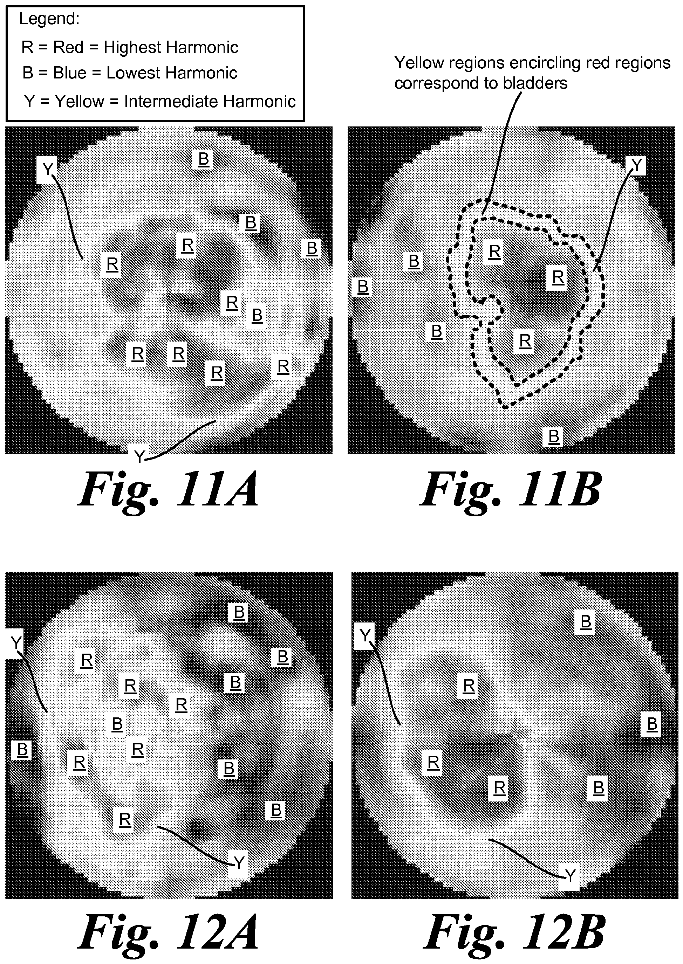

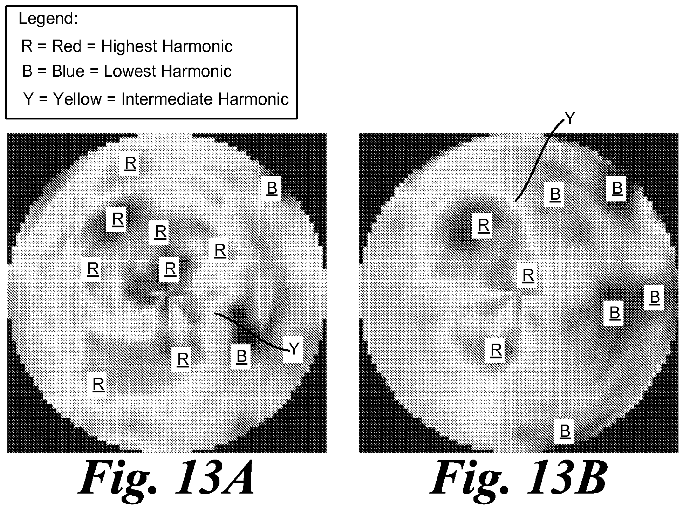

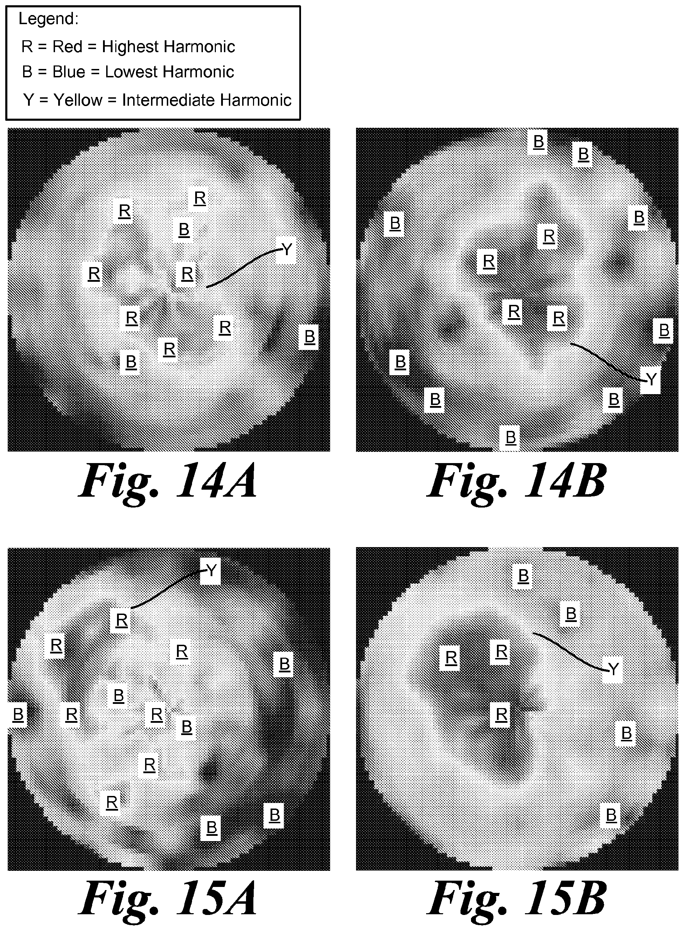

FIGURES 11A-15A illustrates a series of 2D plot of the FFT processed second harmonics and illustrates a diffusely appearing bladder which is hard to delineate and irregular and diffuse shapes presents greater difficulties to estimate bladder volume. The color legend in this figure set denotes red to be the highest harmonic, blue to be the lowest harmonic, and yellow to have a harmonic value of intermediate magnitude. The yellow regions encircling the red regions correspond to bladders. In these FFT processed harmonics, blue like regions appear within the red bladder regions, presenting gaps or other discontinuities within the expected bladder regions.

FIGURES 11B-15B illustrates the respective HAK companions of FIGURES 11A-15A in which the series of 2D plots are HAK processed second harmonics and illustrates a more delineated and compact appearing bladder regions that lends itself to regular shapes that can allow more accurately determined bladder volumes. The color legend in this figure set denotes red to be the highest harmonic, blue to be the lowest harmonic, and yellow regions encircling red regions to correspond to bladders. In FIGURE 11B, a relatively thicker or wider yellow band is seen circumscribing around a centrally located red region. This more pronounced yellow pathway region or band envelops the more central and continuously located red region to denote with more certainty the location of the bladder. In general the HAK processing shows significant improvement in harmonic ratio estimations over that harmonics that are just FFT processed. There are virtually no blue like gaps in the HAK detected and centrally located red regions where the expected bladder regions exhibit a contiguous presence.

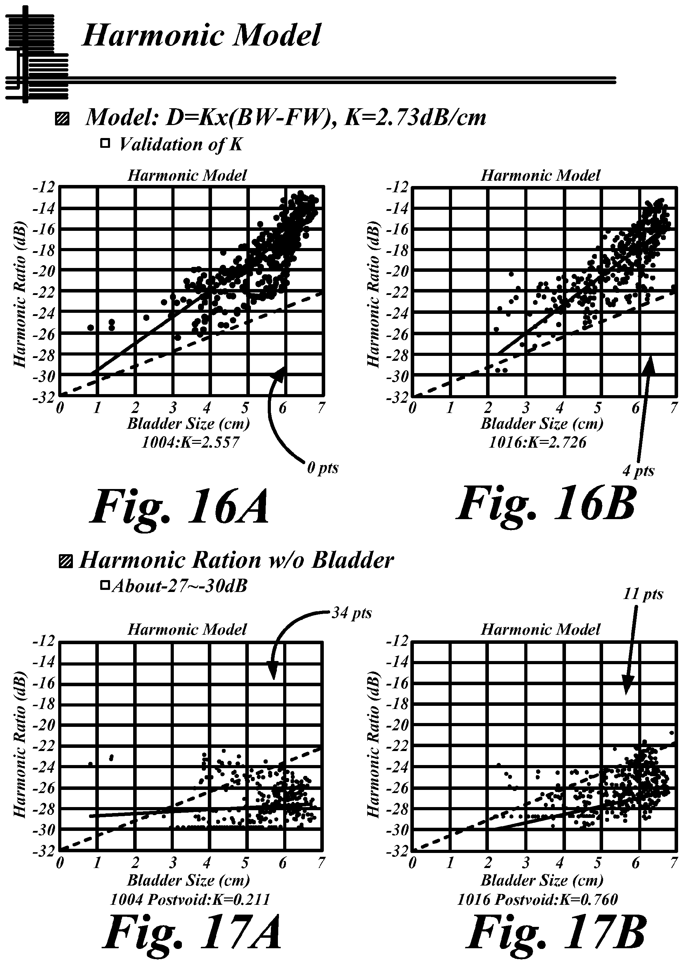

FIGURES 16A-B illustrate regression analyses of harmonic ratio vs. bladder size plots of bladders not voided of urine. Filled bladders exhibit a minimum of data outliers.

FIGURES 17A-B illustrate regression analyses of harmonic ratio vs. bladder size plots in of bladders after voiding of urine. Empty bladders exhibit more data outliers than the filled bladders of FIGURES 16A-B.

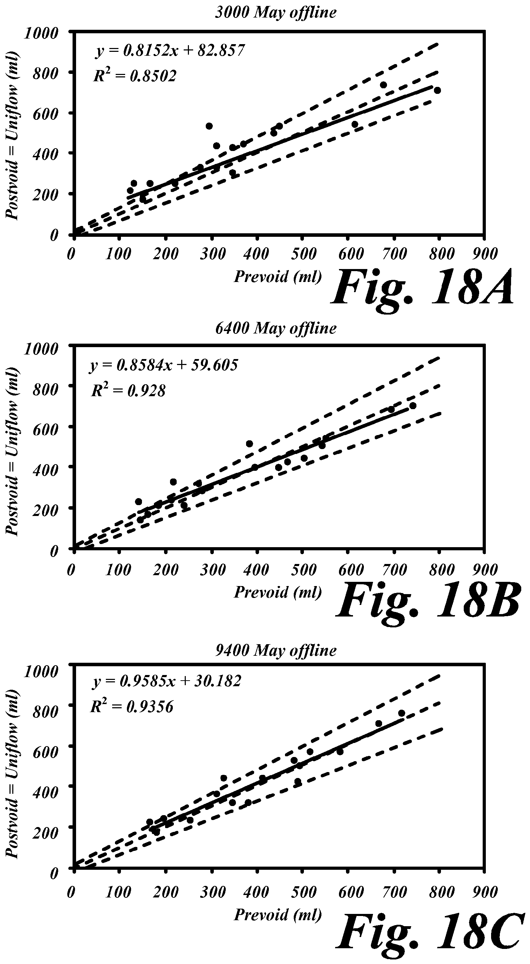

FIGURES 18A-C illustrate regression analysis of measured urine volume vs. HAK algorithm predicted volumes of a clinical group comprising 8 males and ten females. The slopes vary between 0.81 and 0.96 with strong correlation coefficients R² varying between 0.85 and approximately 0.94.

The scope of the invention is not limited by the disclosure of the preferred embodiment. For example, gelatinous masses may be used to modify synthetic tissue and combination fluid and tissue to further define and optimize the sub-aperture neural network algorithm.

## Claims

1. A system (70) to improve image clarity in ultrasound images comprising:
an ultrasound transceiver (10A, 10B, 10C) capable of transmitting ultrasound signals and receiving ultrasound energy returning from a targeted region of interest and producing a plurality of echoic signals; and
a computer (74, 52) having a display (76, 54), the computer (74, 52) having an executable algorithm configured to image (77C) the targeted region-of-interest from the echoic signals using a first harmonic and a second harmonic of the ultrasound energy;
wherein:
the display (76, 54) is further configured to present segmentation of boundary interfaces within the targeted region of interest; and
**characterised in that** the executable algorithm includes a Harmonic Analysis Kernel that includes a window process, a Fast Fourier Transform process, an average process, a normalization of intensity process, a compensation by depth process, and a harmonic smoothing process to generate a harmonic value.

2. The system of claim 1, wherein the image of the region-of-interest is based on the harmonic value.

3. The system of claim 2, wherein the graphic includes a coded map of the region of interest.

4. The system of claim 3, wherein the coded map of the region of interest includes colors assigned to the magnitude of the harmonic value.

5. A method to improve image clarity in ultrasound images from an ultrasound transducer (10A, 10B, 10C) comprising:
transmitting ultrasound energy to a region-of-interest of a subject;
collecting ultrasound echoes returning from the region-of-interest and converting to echoic signals;
processing the echoic signals using a computer executable non-parametric algorithm to generate signal related information which includes a first harmonic and a second harmonic of the ultrasound energy;
generating an image of the boundary surface interfaces within the region-of-interest using the signal related information; and
presenting segmentation of boundary interfaces within the targeted region of interest
**characterised in that** the using the non-parametric executable algorithm includes applying a Harmonic Analysis Kernel that includes executing instructions for window processing, Fast Fourier Transform processing, average processing, normalization of intensity processing, compensation by depth processing, and harmonic smoothing processing to produce a harmonic value.

6. The method of claim 5, wherein imaging of the region of interest includes presenting a coded depiction of the region-of-interest by the magnitude of the harmonic value.

7. The method of claim 6, wherein presenting the coded depiction of the region-of-interest includes color-coding.

## Patentansprüche

1. System zur Verbesserung der Bildschärfe in Ultraschallbildern, umfassend:
einen Ultraschall-Sender-Empfänger (10A, 10B, 10C), der geeignet ist, Ultraschallsignale auszusenden und Ultraschallenergie zu empfangen, die von einer interessierenden Zielregion zurückkehrt und eine Vielzahl von Echosignalen erzeugt; und
einen Computer (74, 52) mit einer Anzeige (76, 54), wobei der Computer (74, 52) einen ausführbaren Algorithmus aufweist, der konfiguriert ist, die interessierende Zielregion aus den Echosignalen unter Anwendung einer ersten Harmonischen und einer zweiten Harmonischen der Ultraschallenergie abzubilden;
worin:
die Anzeige (76, 54) ferner konfiguriert ist, eine Segmentierung von Begrenzungsgrenzflächen innerhalb der interessierenden Zielregion darzustellen; und
**dadurch gekennzeichnet ist, dass**
der ausführbare Algorithmus einen Harmonische-Analysebetriebssystemkern umfasst, der einen Fensterprozess, einen Schnelle-Fourier-Transformations-Prozess, einen Mittelwertprozess, eine Intensitätswertprozessnormierung, einen Tiefenkompensationsprozess und einen Harmonischen-Glättungsprozess zur Erzeugung eines Harmonischen-Werts umfasst.

2. System nach Anspruch 1, worin die Abbildung der interessierenden Region auf dem Harmonischen-Wert basiert.

3. System nach Anspruch 2, worin die graphische Darstellung eine kodierte Karte der interessierenden Region umfasst.

4. System nach Anspruch 3, worin die kodierte Karte der interessierenden Region Farben umfasst, die der Größe des Harmonischen-Wertes zugeordnet sind.

5. Verfahren zur Verbesserung der Bildschärfe in Ultraschallbildern aus einem Ultraschallwandler (10A, 10B, 10C), umfassend:
Aussenden von Ultraschallenergie an eine interessierende Region einer Zielperson;
Auffangen von Ultraschallechos, die von der interessierenden Region zurückkehren und sich in Echosignale umwandeln;
Verarbeiten der Echosignale unter Anwendung eines computer-ausführbaren nicht-parametrischen Algorithmus, um Signal-bezogene Informationen zu erzeugen, die eine erste Harmonische und eine zweite Harmonische der Ultraschallenergie umfassen;
Erzeugen eines Abbildes der Begrenzungsgrenzflächen innerhalb der interessierenden Region unter Anwendung der Signal-bezogenen Informationen; und
Darstellen einer Segmentierung von Begrenzungsgrenzflächen innerhalb der interessierenden Region,
**dadurch gekennzeichnet, dass**
die Anwendung des nicht-parametrischen ausführbaren Algorithmus das Anwenden eines Harmonische-Analysebetriebssystemkerns umfasst, der das Ausführen von Befehlen für ein Fensterverarbeiten, ein Schnelles-Fourier-Transformations-Verarbeiten, ein Mittelwertverarbeiten, eine Intensitätsverarbeitungsnormierung, eine Tiefenkompensationsverarbeitung und ein Harmonisches-Glättungsverarbeiten umfasst, um einen Harmonischen-Wert zu erzeugen.

6. Verfahren nach Anspruch 5, worin das Abbilden der interessierenden Region das Darstellen einer kodierten Abbildung der interessierenden Region durch die Größe des Harmonischen-Wertes umfasst.

7. Verfahren nach Anspruch 6, worin das Darstellen der kodierten Abbildung der interessierenden Region ein Farbkodieren umfasst.

## Revendications

1. Système (70) destiné à améliorer une clarté d'image dans des images ultrasonores, comprenant :
un émetteur-récepteur à ultrasons (10A, 10B, 10C) apte à transmettre des signaux ultrasonores et à recevoir de l'énergie ultrasonore revenant d'une zone d'intérêt ciblée et produisant une pluralité de signaux de résonance ; et
un ordinateur (74, 52) présentant un affichage (76, 54), l'ordinateur (74, 52) présentant un algorithme exécutable configuré de manière à imager (77C) la zone d'intérêt ciblée à partir des signaux de résonance, en utilisant une première harmonique et une seconde harmonique de l'énergie ultrasonore ;
dans lequel :
l'affichage (76, 54) est en outre configuré de manière à présenter une segmentation d'interfaces limites dans la zone d'intérêt ciblée ; et
**caractérisé en ce que**
l'algorithme exécutable inclut un noyau d'analyse d'harmoniques qui inclut un processus de fenêtrage, un processus de transformée de Fourier rapide, un processus de calcul de moyenne, un processus de normalisation d'intensité, un processus de compensation par profondeur, et un processus de lissage d'harmoniques pour générer une valeur harmonique.

2. Système selon la revendication 1, dans lequel l'image de la zone d'intérêt est basée sur la valeur harmonique.

3. Système selon la revendication 2, dans lequel le graphique inclut une carte codée de la zone d'intérêt.

4. Système selon la revendication 3, dans lequel la carte codée de la zone d'intérêt inclut des couleurs affectées à la grandeur de la valeur harmonique.

5. Procédé d'amélioration de clarté d'image dans des images ultrasonores à partir d'un transducteur ultrasonore (10A, 10B, 10C), comprenant les étapes ci-dessous consistant à :
transmettre de l'énergie ultrasonore à une zone d'intérêt d'un sujet ;
collecter des échos ultrasonores revenant de la zone d'intérêt et les convertir en signaux de résonance ;
traiter les signaux de résonance en utilisant un algorithme non paramétrique exécutable par ordinateur en vue de générer des informations connexes au signal qui incluent une première harmonique et une seconde harmonique de l'énergie ultrasonore ;
générer une image des interfaces de surfaces limites dans la zone d'intérêt en utilisant les informations connexes au signal ; et
présenter une segmentation d'interfaces de surfaces limites dans la zone d'intérêt ciblée ;
**caractérisé en ce que**
l'utilisation de l'algorithme exécutable non paramétrique inclut l'application d'un noyau d'analyse d'harmoniques qui inclut l'exécution d'instructions pour un processus de fenêtrage, un processus de transformée de Fourier rapide, un processus de calcul de moyenne, un processus de normalisation d'intensité, un processus de compensation par profondeur et un processus de lissage d'harmoniques en vue de produire une valeur harmonique.

6. Procédé selon la revendication 5, dans lequel l'étape d'imagerie de la zone d'intérêt inclut la présentation d'une représentation codée de la zone d'intérêt par la grandeur de la valeur harmonique.

7. Procédé selon la revendication 6, dans lequel l'étape de présentation de la représentation codée de la zone d'intérêt inclut un codage couleur.
